# EUROPÄISCHE PATENTSCHRIFT

(11) **EP 1 257 309 B1**
(45) Veröffentlichungstag und Bekanntmachung des Hinweises auf die Patenterteilung: **30.05.2007**
(21) Anmeldenummer: 01902236.7
(22) Anmeldetag: 13.02.2001
(51) Int. Cl.: A61M 5/315

(54) **ZWEIKOMPONENTIGER KOLBENSTOPFEN**
PLUNGER PLUG CONSISTING OF TWO COMPONENTS
BOUCHON DE PISTON CONSTITUE DE DEUX COMPOSANTS

(30) Priorität: 15.02.2000 DE 10006560
(43) Veröffentlichungstag der Anmeldung: 20.11.2002
(73) Patentinhaber: TecPharma Licensing AG, 3401 Burgdorf (CH)
(72) Erfinder: KIRCHHOFER, Fritz, CH-3454 Sumiswald (CH); HIRSIGER, Michel, CH-3032 Hinterkappelen (CH); SCHIFFMANN, Frank, CH-3400 Burgdorf (CH); STOLLER, Hanspeter, CH-3012 Bern (CH)
(74) Vertreter: Cremer, Ulrike Theresia
(86) Internationale Anmeldenummer: PCT/CH2001/000096
(87) Internationale Veröffentlichungsnummer: WO 2001/060434

(56) Entgegenhaltungen:
- EP-A- 0 925 798
- EP-A- 1 099 449
- CH-A- 268 694
- FR-A- 1 006 965
- US-A- 5 226 897
- US-A- 5 397 313
- US-A- 5 902 276
- US-A- 5 997 511
- US-A- 6 004 300

## Beschreibung

Die Erfindung betrifft einen Kolbenstopfen für einen Medikamenteninjektor sowie ein Verfahren zur Herstellung eines solchen Kolbenstopfens.

Medikamenteninjektoren weisen üblicherweise einen zylindrischen Aufnahmebehälter für ein flüssiges Medikament auf, welches mittels eines Kolbens durch eine Kanüle ausgespritzt wird. Diese Kolben haben einen Schaft sowie einen Kolbenstopfen, der im Zylinder gegen dessen Innenwand dicht abschließt. Hierzu werden herkömmlicherweise Kolben verwendet, die mit mindestens einem in einer Umfangsnut positionierten Dichtring versehen sind. Bei bekannten Ausführungsformen besteht der Kolbenstopfen aus einem relativ hartem Material und die Dichtringe, die zur besseren Abdichtung aus einem weicheren Material bestehen, werden gummiartig dehnbar in Umfangsnuten eingesetzt. Sie werden also lediglich durch ihre eigene innere Spannkraft in den Nuten gehalten.

Die Montage der Dichtringe bei der obigen herkömmlichen Ausführungsform muss in einem separaten Schritt durchgeführt werden und ist deshalb zeitaufwändig. Auch ist der Halt der Dichtringe in den Umfangsnuten stark von der Federelastizität der Dichtringe abhängig, so dass schlecht gefertigte Dichtringe oder solche, die aus einem schon etwas älterem Material hergestellt sind, leicht aus den Nuten herausrutschen. Weil die Dichtringe einfach in die Nuten eingespannt werden, besteht grundsätzlich immer die Gefahr, dass sie diese Halterung verlassen oder sich in der Halterung beim Gebrauch stark verwinden, wodurch größere Dichtigkeitsprobleme entstehen.

Man hat versucht, dieses Problem dadurch zu lösen, dass Kolbenstopfen als ganze Bauteile aus einem weichen Vollgummimaterial hergestellt wurden, wobei die Dichtringe lediglich als vorstehende Umfangsansätze des Stopfenmaterials ausgebildet wurden. Auch diese Lösung birgt jedoch Probleme in sich, speziell bei einem hohen Gegendruck durch die auszutreibende Flüssigkeit. Ein solcher hoher Gegendruck kann beispielsweise bei einer Verformung oder bei einem Abknicken der Kanüle entstehen. Wegen des insgesamt weichen Stopfenmaterials wird dieses in solchen Fällen nachgeben und der Kolben fährt weiter nach vorne in Austrittsrichtung, wobei die Flüssigkeit zwischen Kolben und Zylinderinnenwand vorbei nach hinten gedrückt wird. Insbesondere bei der Verwendung von nach aussen abgeschlossenen Injektoren, beispielsweise sogenannten "Pens" besteht hierbei die Möglichkeit, dass der Patient diesen Vorgang gar nicht bemerkt und glaubt, er hätte sich das Medikament injiziert, obwohl es am Kolben vorbei in den hinteren Innenraum des Zylinders geströmt ist.

Ferner weisen Kolbenstopfen gemäss dem Stand der Technik gewöhnlich einfach in den Stopfenkörper eingeformte Gewindeabschnitte auf, die keine optimale Verbindung zu einem Stössel herstellen.

In der CH 268694 ist beispielsweise ein Spritzenzylinder und Kolbenstopfen aus Glas und eine Kolbenstange aus Metall beschrieben. Der Kolbenstopfen liegt mit geringem Spiel an der Innenwandung des Zylinders an. Zwischen dem Kolbenstopfen und der Zylinderwand ist ein Dichtring vorgesehen. An seiner rückwärtigen Stirnfläche weist der Kolbenstopfen eine Bohrung auf, in welche die Kolbenstange teilweise eingesetzt Ist. Ein weiteres Beispiel für einen Kolbenstopfen ist aus der US 5.902,276 bekannt. Dort wird ein Kolbenstopfen beschrieben, der einen Hartplastikkem aufweist, welcher an seinem distalen Ende einen Gummiüberzug aufweist. Der Gummiüberzug umgibt das distale Ende des Stopfens und bildet eine Dichtung mit einer Innenwand eines Spritzenkörpers. Am proximalen Ende weist der Kolbenstopfen einen Fortsatz auf, an welchen eine Kolbenstange angekoppelt werden kann.

Es ist die Aufgabe der vorliegenden Erfindung einen Kolbenstopfen für einen Medikamenteninjektor sowie ein Verfahren zu dessen Herstellung bereitzustellen, um die obigen Nachteile des Standes der Technik zu überwinden, Insbesondere soll ein Kolbenstopfen bereitgestellt werden, der einen optimierten Eingriff mit den an ihn anschliessenden Bauteilen gestattet. Er soll speziell eine optimierte Dichtheit liefern und in einfacher Weise fertigzustellen sein bzw. eine optimierte Verbindung zu einem einzuschraubenden Stössel aufweisen.

Diese Aufgabe wird durch einen Kolbenstopfen für einen Medikamenteninjektor gelöst, der einen Stopfenkörper, der einen Eingriffsabschnitt, insbesondere Gewindeeingriffsabschnitt für einen Stössel aufweist, und mindestens ein, zumindest teilweise umfänglich an dem Stopfenkörper positioniertes Dichtelement aufweist, wobei der Eingriffsabschnitt, insbesondere Gewindeeingriffsabschnitt, stoffschlüssig mit dem Stopfenkörper verbunden ist. Dabei besteht der Stopfenkörper aus einem Thermoplasten und der Eingriffsabschnitt aus einem weiteren Thermoplasten.

Das Dichtelement kann ein separates oder diskret vorhandenes Dichtelement sein und durch eine stoffschlüssige Verbindung nach seiner Anbringung an dem Stopfenkörper unlösbar mit diesem verbunden werden. Dadurch kann es sich vorteilhafterweise nicht mehr verwinden oder aus seiner Positionierungseinnchtung austreten, was Dichtigkeitsprobleme, die gemäss dem Stand der Technik durch solche Schwierigkeiten aufgetreten sind, vermeidet. Ferner ermöglicht eine stoffschlüssige Verbindung am Dichtelement, die Position seiner Dichtfläche akkurat einzuhalten, und bei der Herstellung des Kolbenstopfens kann von einer eindeutigen Position dieser Dichtfläche ausgegangen werden, so dass an der Dichtfläche wirksame Verbesserungen, zum Beispiel durch spezielle Formgebung realisiert werden können.

Dabei bleibt immer der Vorteil erhalten, dass der Stopfen aus verschiedenen Materialien hergestellt werden kann, nämlich beispielsweise aus einem harten Material für den Stopfenkörper und einem weichen Material für das Dichtelement bzw. die Dichtelemente. Die Probleme weicher Stopfenkörper, wie sie oben beschrieben wurden, werden demnach vermieden.

Obiges gilt sinngemäss auch für den Eingriff des Stopfenkörpers mit einem Stössel. Der Eingriffsabschnitt, insbesondere Gewindeeingriffsabschnitt für einen solchen Stössel kann nämlich gemäss der Erfindung stoffschlüssig mit dem Stopfenkörper verbunden sein. Insbesondere besteht hierdurch die Möglichkeit durch Auswahl eines geeigneten Materials für den Eingriffsabschnitt mit geeigneter Härte oder Weichheit einen optimierten und speziell sicheren bzw. geeignet gedämpften Eingriff mit dem Stössel herzustellen. Die stoffschlüssige Verbindung gestattet auch hier eine zuverlässige Positionsfixierung.

Es ist im Rahmen der Erfindung möglich, ein, zwei oder mehrere Dichtelemente an einem Stopfenkörper zu positionieren. Die Anzahl wird vom jeweiligen Anwendungsfall abhängen, und die Dichtelemente können in Form von Dichtringen in umlaufenden Ausnehmungen, zum Beispiel Nuten, des Stopfenkörpers positioniert werden.

Ferner kann beispielsweise eine Dichtkappe, die eine umfängliche Dichtlippe aufweist, vorne auf den Stopfenkörper aufgesetzt werden. Eine solche Dichtlippe ist dann vorteilhafterweise als ein von der Kappe abstehender Ringvorsprung ausgebildet, der schräg nach vorne, d.h. in Richtung auf das zu verdrängende Flüssigkeitsvolumen vom Kappenkörper abragt.

Bei einer Ausführungsform der Erfindung wird das Dichtelement und/oder der Eingriffsabschnitt, insbesondere Gewindeeingriffsabschnitt an seiner Kontaktfläche mit dem Stopfenkörper angeklebt, während alternativ ein Dichtelement und/oder ein Eingriffsabschnitt, insbesondere Gewindeeingriffsabschnitt vorgesehen werden kann, der an seiner Kontaktfläche mit dem Stopfenkörper integral angeformt ist. Mit integraler Anformung ist hierbei gemeint, dass die Materialien des Dichtelementes sowie des Stopfenkörpers zu einem durchgehenden Material zusammengebracht werden, und dies kann beispielsweise dadurch erzielt werden, dass das Dichtelement und/oder der Eingriffsabschnitt, insbesondere Gewindeeingriffsabschnitt an der Kontaktfläche mit dem Stopfenkörper verschmolzen wird.

Eine solche Verschmelzung bzw. integrale Anformung erfolgt vorzugsweise im Zweikomponenten-Spritzguss, wobei das Dichtelement und/oder der Eingriffsabschnitt, insbesondere Gewindeeingriffsabschnitt an die Kontaktfläche mit dem Stopfenkörper angespritzt wird. Durch die dabei entstehende Erwärmung der Kontaktfläche kann hierbei eine stoffschlüssige Verschmelzung sichergestellt werden.

Der Stopfenkörper wird gemäss der Erfindung aus einem Thermoplasten, insbesondere einem relativ harten, nicht elastomeren Thermoplasten, bevorzugt aus Propylen ausgebildet, und der Eingriffsabschnitt, insbesondere Gewindeeingriffsabschnitt wird vorzugsweise ebenfalls aus einem Thermoplasten, jedoch insbesondere einem relativ weichen, elastomeren Thermoplasten ausgebildet, bevorzugt aus Santoprene.

Um die Verschmelzung zu fördern bzw. zu vereinfachen, werden gemäss einer Ausführungsform der Erfindung Materialien für den Stopfenkörper und das Dichtelement und/oder den Eingriffsabschnitt, insbesondere Gewindeeingriffsabschnitt verwendet, deren Schmelzpunkte nicht weiter als 35°C, insbesondere nicht weiter als 20°C bis 25°C auseinander liegen.

Gemäß einer bevorzugten Ausgestaltung des Kolbenstopfens bildet das Dichtelement, wenn es als Dichtring ausgebildet ist, im Bereich seiner äußeren Dichtfläche eine Zunge aus, um die Flüssigkeitsabdichtung beim Ausspritzen der Flüssigkeit weiter zu erhöhen. Von besonderem Vorteil ist es hierbei, wenn die Dichtzunge an der Außenseite einer konkaven vorderen Ausnehmung angeordnet ist, wobei die Vorderseite als die Seite anzusehen ist, die der Flüssigkeit im Injektorzylinder zugewandt ist.

Der erfindungsgemäße Kolbenstopfen und das Verfahren zu seiner Herstellung sind in den Ansprüchen beschrieben. Im Weiteren wird zur detaillierten Beschreibung der Erfindung auf die beiliegenden Figuren Bezug genommen. Es zeigen:
- Figur 1: einen ausschnittsweise vergrößerten Bereich eines Kolbenstopfens mit einem Dichtring; und
- Figur 2: den Kolbenstopfen gemäß Figur 1 in einer Gesamtansicht in einem Injektorzylinder;
- Figur 3: eine Darstellung gemäß Figur 1 für einen Kolbenstopfen mit einem speziellen Dichtring;
- Figur 4: eine Darstellung wie in Figur 2 für den Kolbenstopfen mit dem speziellen Dichtring der Figur 3;
- Figuren 5a und 5b: eine weitere Ausführungsform eines Kolbenstopfens mit einer Dichtkappe, die eine umlaufende Dichtzunge aufweist;
- Figuren 6 und 7: den Figuren 1 und 2 bzw. 3 und 4 entsprechende Darstellungen für einen Kolbenstopfen gemäß dem Stand der Technik; und
- Figur 8: eine Ausführungsform eines erfindungsgemäßen Kolbenstopfens mit stoffschlüssig verbundenem Gewindeeingriffsabschnitt.

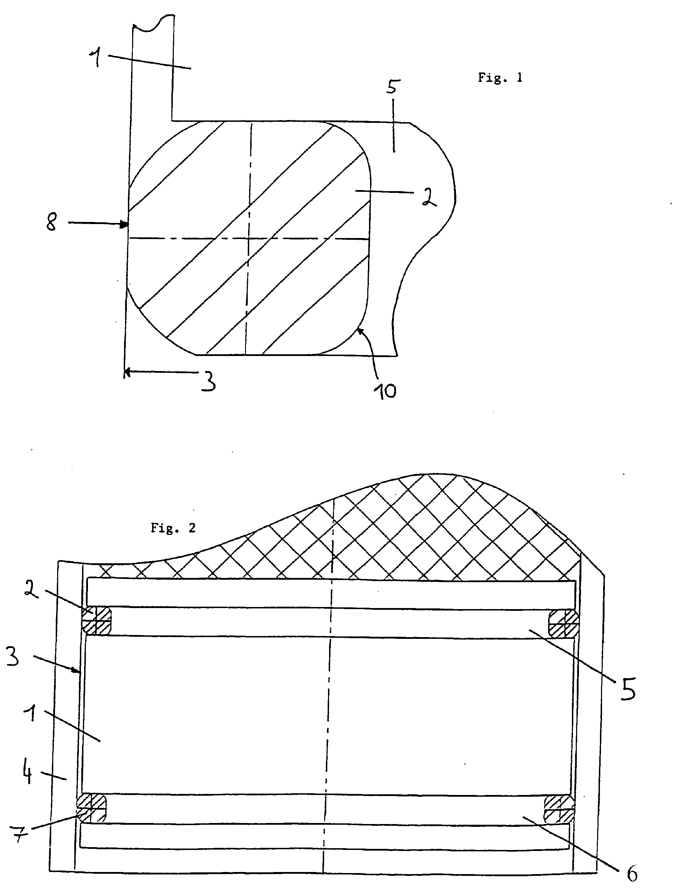

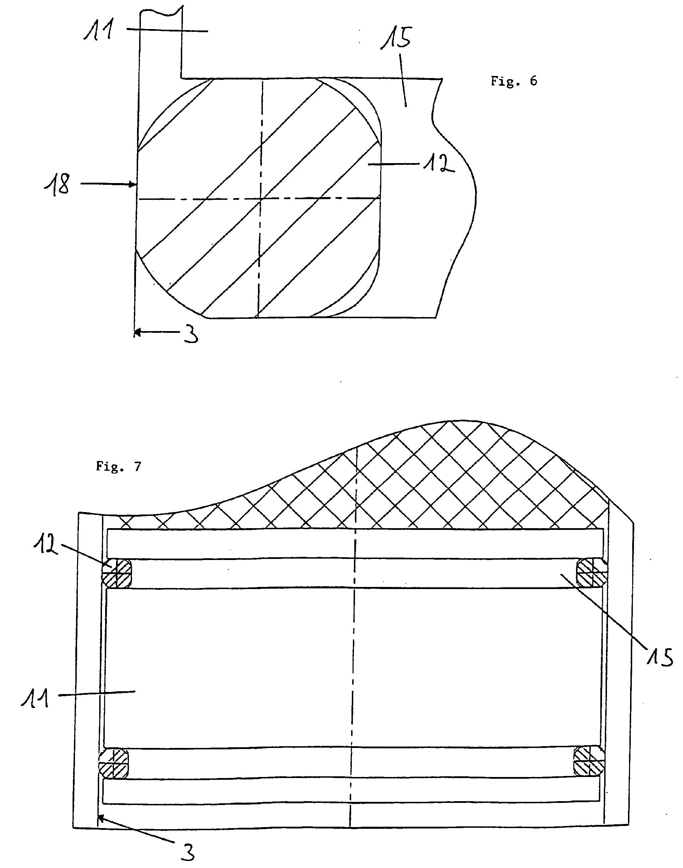

Ein herkömmlicher Kolbenstopfen mit einem lose in einer Nut sitzenden Dichtring ist in den Figuren 6 und 7 gezeigt. Der Kolbenstopfen 11, der wie in Figur 7 gezeigt ist, in einem Glaskolben sitzt, weist die Nut 15, in welche der Dichtring 12 lose eingesetzt ist. In der Nut 15 wird der Dichtring 12 lediglich durch seine innere Spannkraft gehalten, jedoch entstehen, wie insbesondere aus Figur 6 hervorgeht, auch im Bereich der Kontaktflächen zwischen dem Kolben 11 bzw. dem Inneren der Nut 15 noch Zwischenräume. Der lose Sitz des Ringes 12 gestattet eine Verformung, Verwindung und möglicherweise sogar einen Austritt des Ringes 12 aus der Nut 15 und bringt damit Dichtigkeitsprobleme mit sich. Bei einer Verformung kann auch die Kontaktfläche 18, an der der Ring 12 mit der Innenwand 3 des Zylinders in Kontakt kommt, nicht mehr in ihrer ursprünglichen Form aufrechterhalten werden, und vor allem ist sie durch die Bewegungsmöglichkeiten des Ringes 12 nicht eindeutig am Ring definiert. Eine Art einer verbesserten Ausführung des Kolbenstopfens ist in den Figuren 1 und 2 gezeigt, wobei die Figur 1 einen Bereich um einen Dichtring 2 herum in Vergrößerung der Figur 2 zeigt.

Der Kolbenstopfen besteht aus dem Stopfenkörper 1, der zwei Umfangsnuten 5 und 6 aufweist. In diese Umfangsnuten sind die Dichtringe 2 und 7 positioniert, und zwar in stoffschlüssiger Verbindung mit dem Material des Stopfenkörpers 1 bzw. der Nuten 5 und 6. An der Kontaktfläche 10 (Figur 1) ist der Ring 2 durch Ankleben oder durch Verschmelzen stoffschlüssig und unlösbar mit dem Material des Stopfenkörpers verbunden. Damit wird verhindert, dass sich der Ring 2, 7 in der Nut 5, 6 verwindet, bewegt oder diese zum Teil oder ganz verlässt. Er bleibt somit erstens formfest und zweitens lagefest gegenüber dem Stopfenkörper 1 und kann damit seine Dichtungsaufgaben wesentlich besser erfüllen als ein lose eingesetzter Ring, wie er beim Kolbenstopfen gemäß dem Stand der Technik verwendet wird (Figuren 6 und 7).

Insbesondere durch die Lagefestigkeit gibt sich die bessere Abdichtung schon dadurch, dass die Kontaktfläche 8, an der Kolben die Innenwand 3 des umgebenden Zylinders 4 kontaktiert, eine definierte und gleichbleibende Fläche ist, deren Gestalt sich beim Verschiebungsvorgang des Kolbenstopfens nur in sehr geringem Maße ändern wird. Auch aus diesem Umstand heraus wird die Dichtigkeit des Kolbenstopfens garantiert.

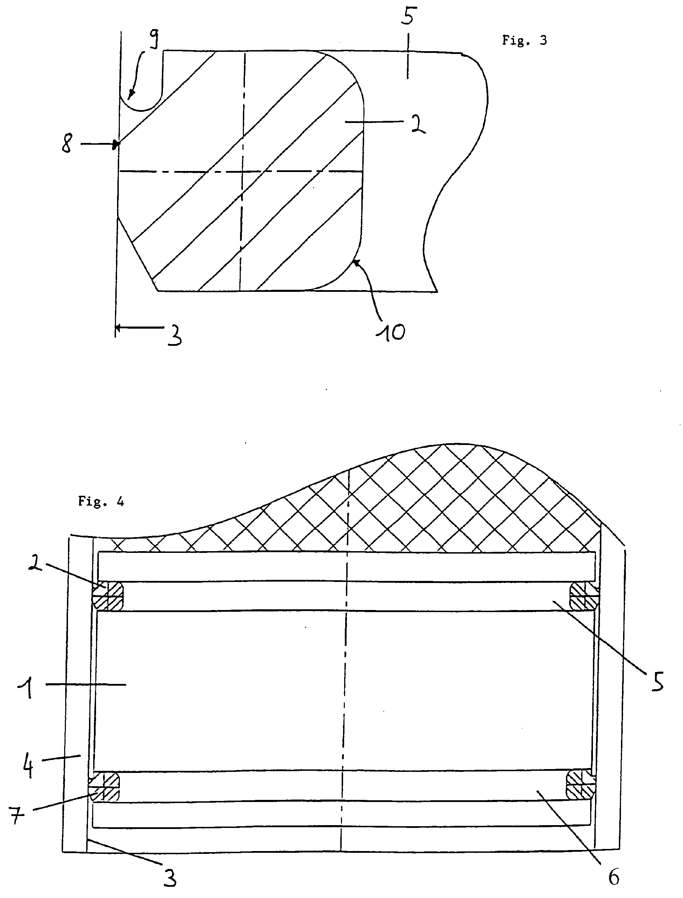

Der letztgenannte Vorteil stellt ferner die Möglichkeit zur Verfügung, den Dichtring im Bereich seiner Kontaktfläche mit der Zylinderinnenwand 3 in speziell vorteilhafter Weise auszugestalten, und eine solche bevorzugte Ausführungsform ist in den Figuren 3 und 4 dargestellt, welche ansichtsweise den Figuren 1 und 2 entsprechen. Bis auf den Bereich der Kontaktfläche 8 des Dichtrings 2 mit der Innenwand 3 des Zylinders 4 entspricht diese Ausführungsform derjenigen gemäss den Figuren 1 und 2, und auf die entsprechende Beschreibung wird hingewiesen.

Wie erwähnt unterscheidet sich die Ausführungsform gemäss den Figuren 3 und 4 in Hinsicht auf den Dichtring 2 von der vorherigen Ausführungsform, und zwar speziell im Bereich der Kontaktfläche 8 zwischen der Innenwand 3 des Zylinders 4 und dem Dichtring. In diesem Bereich ist an der Vorderseite (in Figur 3 oben) des Dichtringes eine konkave Ausnehmung vorgesehen, die im Wandbereich eine Dichtzunge 9 ausbildet. Diese Dichtzunge liegt mit ihrer Spitze an der Wand 3 an und lenkt beim Vorschieben des Kolbenstopfens die Flüssigkeit durch den konkaven Bereich zur Innenseite hin ab, so dass mit dieser Ausführungsform noch besser verhindert werden kann, dass Flüssigkeit zwischen den Dichtring 2 und die Innenwand 3 gerät, wenn der Kolbenstopfen vorgeschoben wird. Dies trägt zur weiteren Verbesserung der Dichtigkeit bei.

Es ist noch anzumerken, dass bei allen Ausführungsformen der Vorteil besteht, dass der Stopfenkörper 1 selbst aus einem relativ harten Material hergestellt werden kann, so dass Probleme, wie sie bei weichen Vollgummistopfen mit angeformten Dichtansätzen entstehen und oben beschrieben wurden, nicht auftreten während trotzdem ein weiches Material für den Abdichtungsbereich verwendet werden kann.

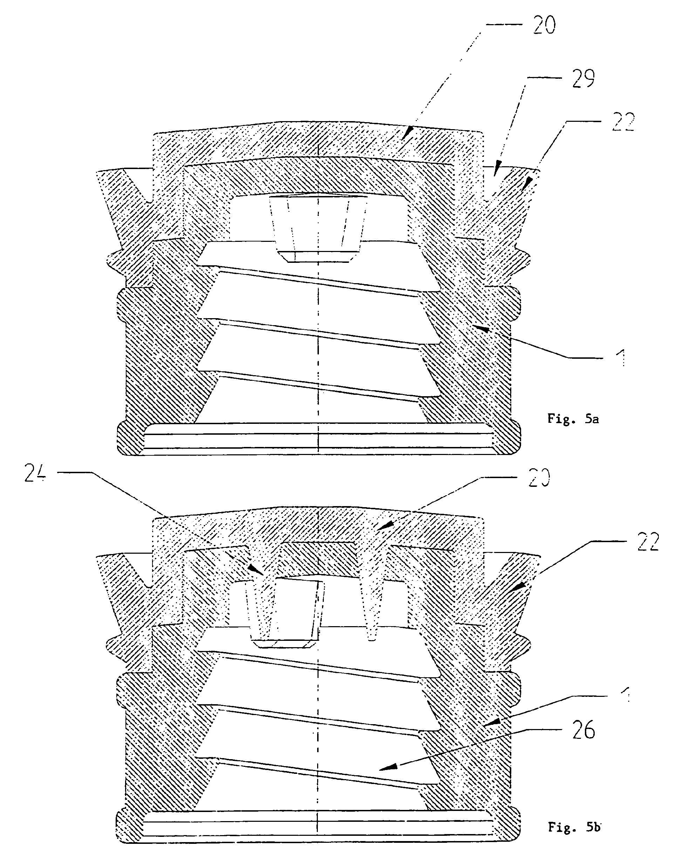

Eine weitere Ausführungsform eines Kolbenstopfens ist den Schnittansichten zu entnehmen, die in den Figuren 5a und 5b dargestellt sind. Diese beiden Figuren 5a und 5b zeigen winkelversetzte Längsschnitte durch denselben Kolbenstopfen, der gemäss dieser Ausführungsform mit einer stoffschlüssig verbundenen Dichtkappe versehen ist.

Hierbei sitzt die Dichtkappe bzw. der Kappenkörper 20 auf dem vorderen Ende des Stopfenkörpers 1, d.h. auf der dem zu verdrängenden Flüssigkeitsvolumen zugewandten Seite. Mittig im Stopfenkörper ist in den Figuren 5a und 5b ein mit 26 bezeichneter Gewindeeingriff für den Stößel des Injektors gezeigt.

Die Dichtkappe 20 in der Ausführungsform gemäß den Figuren 5a und 5b hat durch ihre kappenartige Anbringung eine sehr große Verbindungsfläche mit dem Stopfenkörper 1, so dass nach der Herstellung der stoffschlüssigen Verbindung ein ausgezeichneter Halt der Kappe 20 an dem Stopfenkörper 1 sichergestellt ist. Um diesen Halt noch zu verbessern kann der Kappenkörper 20 noch, wie in Figur 5b gezeigt, mit von der Stirnseite nach innen abstehenden Fortsätzen 24 versehen werden, die in entsprechende Ausnehmungen an der vorderen Stirnseite des Stopfenkörpers 1 eingreifen. Hierdurch wird der Halt noch verbessert, insbesondere, wenn ein Stößel in den Eingriff 26 eingeschraubt und dann die Spitzen der Fortsätze 24 flach verformt werden, so dass sich eine zusätzliche formschlüssige Anbindung ergibt.

Der Kappenkörper 20 ist umfänglich mit der Dichtlippe 22 versehen, die etwa in Höhe seiner Basis schräg nach vorne zum zu verdrängenden Flüssigkeitsvolumen hin abragt. Wie in den Figuren 5a und 5b gezeigt ist, bildet die Außenkante dieser Dichtlippe 22 den radial äußersten Teil des gesamten Kolbenstopfens und liegt damit nach dem Einbringen in einem Zylinder unter Spannung an dessen Innenwand an, wodurch eine hervorragende Abdichtung gewährleistet wird. Ebenso wie bei den Verhältnissen gemäß Figur 3 und Figur 4 wird durch die nach vorne abragende Ausgestaltung der Dichtlippe 22 eine konkave vordere Ausnehmung erzeugt, die beim Verschieben des Kolbenstopfens die Flüssigkeit durch den konkaven Bereich zur Innenseite hin ablenkt und damit eine noch bessere Abdichtung gewährleistet.

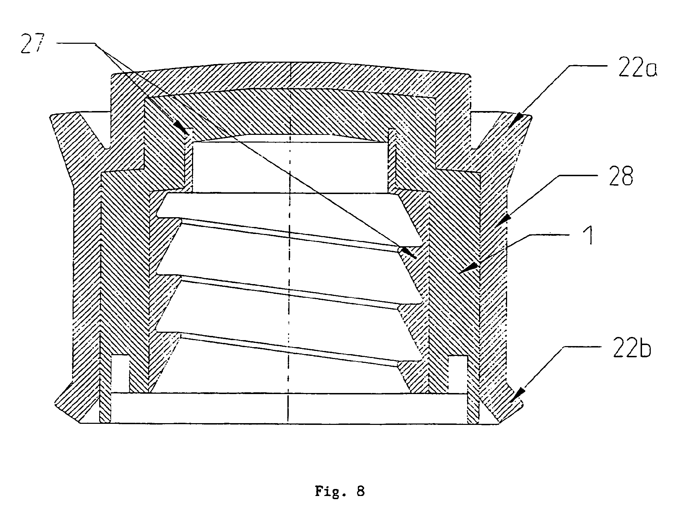

Die Figur 8 zeigt eine weitere Ausführungsform in einer Darstellung, die im Wesentlichen derjenigen in den Figuren 5a und 5b entspricht. Der hier gezeigte Stopfenkörper 1 weist an seiner Außenseite eine stoffschlüssig verbundene Dichtkappe 28 auf, die im vorliegenden Fall den gesamten Stopfenkörper 1 bis mit zu seiner Basis hin übergreift und dabei zwei Dichtlippen 22a und 22b ausbildet.

Die vorliegende Erfindung wird in der Figur 8 deutlich, und zwar bei Betrachtung des hier mit dem Bezugszeichen 27 bezeichneten Gewindeeingriffsabschnittes für einen Stößel. Bei der Ausführungsform nach Figur 8 ist nämlich dieser Gewindeeingriffsabschnitt 27 ebenfalls aus einem anderen, nämlich einem weicheren Material gefertigt als der Stopfenkörper 1, und er ist an seiner äußeren Kontaktfläche mit dem Stopfenkörper 1 stoffschlüssig mit diesem verbunden. Hierdurch ergibt sich erfindungsgemäß die Möglichkeit, auch diesen Eingriffspunkt des Kolbenstopfens mit einem an ihm anliegenden Bauteil zu optimieren. Durch die stoffschlüssige Verbindung des separaten oder diskret vorhandenen Gewindeeingriffsabschnittes 27 kann sichergestellt werden, dass dieses seine Position akkurat und genau einhält, wobei die Möglichkeit besteht, durch Auswahl eines geeigneten Materials für den Eingriffsabschnitt einen sicheren und geeignet gedämpften Eingriff mit dem Stößel herzustellen.

## Patentansprüche

1. Kolbenstopfen für einen Medikamenteninjektor mit einem Stopfenkörper (1), der einen Eingriffsabschnitt (26, 27) für einen Stössel aufweist und mit mindestens einem zumindest teilweise umfänglich an dem Stopfenkörper (1) positionierten Dichtelement (2, 7, 20), wobei der Eingriffsabschnitt (26, 27) stoffschlüssig mit dem Stopfenkörper (1) verbunden ist, **dadurch gekennzeichnet, dass** der Eingriffsabschnitt aus einem weicheren Material besteht als der Stopfenkörper.

2. Kolbenstopfen nach Anspruch 1, **dadurch gekennzeichnet, dass** der Stopfenkörper (1) aus einem relativ harten nicht elastomeren Thermoplasten und der Eingriffsabschnitt (26, 27) aus einem relativ weichen elastomeren Thermoplasten besteht.

3. Kolbenstopfen nach Anspruch 1 oder 2, **dadurch gekennzeichnet, dass** der Stopfenkörper (1) aus Polypropylen und der Eingriffsabschnitt (26, 27) aus Santoprene besteht.

4. Kolbenstopfen nach einem der vorhergehenden Ansprüche, **dadurch gekennzeichnet, dass** der Eingriffsabschnitt (26, 27) an seiner Kontaktfläche mit dem Stopfenkörper (1) verklebt, integral angeformt oder verschmolzen, bzw. im 2-Komponenten-Spritzguss an die Kontaktfläche angespritzt ist.

5. Verfahren zur Herstellung eines Kolbenstopfens für einen Medikamenteninjektor, bei dem ein Stopfenkörper (1) mit einem Eingriffsabschnitt (26, 27) für einen Stössel ausgebildet wird und mindestens ein Dichtelement (2, 7, 20) zumindest teilweise umfänglich an dem Stopfenkörper (1) positioniert wird, wobei der Eingriffsabschnitt aus einem weicheren Material besteht als der Stopfenkörper, **dadurch gekennzeichnet, dass** der Eingriffsabschnitt (26, 27) stoffschlüssig mit dem Stopfenkörper (1) verbunden wird.

6. Verfahren nach Anspruch 5, wobei der Stopfenkörper (1) aus einem relativ harten nicht elastomeren Thermoplasten, insbesondere aus Polypropylen, und der Eingriffsabschnitt (26, 27) aus einem relativ weichen elastomeren Thermoplasten, insbesondere Santoprene, bestehen.

7. Verfahren nach Anspruch 5 oder 6, bei dem der Eingriffsabschnitt (26, 27) an seiner Kontaktfläche mit dem Stopfenkörper (1) verklebt oder integral angeformt wird.

8. Verfahren nach Anspruch 7, bei dem der Eingriffsabschnitt (26, 27) an seiner Kontaktfläche mit dem Stopfenkörper (1) verschmolzen bzw. im 2 Komponenten-Spritzguss an die Kontaktfläche mit dem Stopfenkörper (1) angespritzt wird.

9. Verfahren nach einem der Ansprüche 5-8, bei dem zuerst der Stopfenkörper (1) in seine Endform gespritzt und verfestigt wird und danach der Eingriffsabschnitt (26, 27) angespritzt wird, wobei durch eine Erwärmung der Kontaktfläche eine stoffschlüssige Verschmelzung erfolgt.

10. Verfahren nach Anspruch 9, bei dem die Schmelzpunkte des Thermoplasten des Stopfenkörpers (1) und des Thermoplasten des Eingriffsabschnitts (26, 27) nicht weiter als 35° C. insbesondere nicht weiter als 20° C bis 25° C, auseinander liegen.

## Claims

1. Piston stopper for a medicaments injector, with a stopper body (1) which has an engagement section (26, 27) for a plunger and with at least one sealing element (2, 7, 20) positioned at least partly circumferentially on the stopper body (1), wherein the engagement section (26, 27) is connected substance-locking to the stopper body (1), **characterized in that** the engagement section is composed of a softer material than the stopper body.

2. Piston stopper according to claim 1, **characterized in that** the stopper body (1) is composed of a relatively hard non-elastomeric thermoplast and the engagement section (26, 27) from a relatively soft elastomeric thermoplast.

3. Piston stopper according to claim 1 or 2, **characterized in that** the stopper body (1) is composed of polypropylene and the engagement section (26, 27) from santoprene.

4. Piston stopper according to one of the previous claims, **characterized in that** the engagement section (26, 27) is glued, integrally moulded or melted at its contact surface to the stopper body (1), or gated to the contact surface in the 2-component injection moulding.

5. Process for the manufacture of a piston stopper for a medicaments injector, in which a stopper body (1) is formed with an engagement section (26, 27) for a plunger and at least one sealing element (2, 7, 20) is positioned at least partly circumferentially on the stopper body (1), wherein the engagement section is composed of a softer material than the stopper body, **characterized in that** the engagement section (26, 27) is connected substance-locking to the stopper body (1).

6. Process according to claim 5, wherein the stopper body (1) is composed of a relatively hard non-elastomeric thermoplast, in particular of polypropylene, and the engagement section (26, 27) of a relatively soft elastomeric thermoplast, in particular of santroprene.

7. Process according to claim 5 or 6, in which the engagement section (26, 27) is glued or integrally moulded at its contact surface to the stopper body (1).

8. Process according to claim 7, in which the engagement section (26, 27) is melted with the stopper body (1) at its contact surface or gated with the stopper body (1) to the contact surface in the 2 component injection moulding.

9. Process according to one of claims 5-8, in which the stopper body (1) is first injected into its final form and solidified and then the engagement section (26, 27) is gated to it, wherein a substance-locking fusion takes place through a heating of the contact surfaces.

10. Process according to claim 9, in which the melting points of the thermoplast of the stopper body (1) and of the thermoplast of the engagement section (26, 27) are not more than 35°C, in particular not more than 20°C to 25°C, apart.

## Revendications

1. Bouchon de piston pour un injecteur de médicament avec un corps de bouchon (1) qui présente une section d'engrènement (26,27) pour un coulisseau et avec au moins un élément d'étanchéité (2, 7, 20) positionné au moins partiellement sur la circonférence du corps de bouchon (1), la section d'engrènement (26,27) étant refermée par un matériau avec le corps de bouchon (1), **caractérisé en ce que** la section d'engrènement consiste en un matériau plus mou que le corps de bouchon.

2. Bouchon de piston selon la revendication 1, **caractérisé en ce que** le corps de bouchon (1) consiste en un thermoplastique non élastomère relativement dur et la section d'engrènement (26,27) en un thermoplastique élastomère relativement mou.

3. Bouchon de piston selon la revendication 1 ou 2, **caractérisé en ce que** le corps de bouchon (1) consiste en polypropylène et la section d'engrènement (26,27) en santoprène.

4. Bouchon de piston selon l'une des revendications précédentes, **caractérisé en ce que** la section d'engrènement (26,27) est collée avec le corps de bouchon (1) par sa surface de contact, solidarisée intégralement par formage ou fusionnée, ou appliquée par moulage par injection à deux composants sur la surface de contact.

5. Procédé pour la fabrication d'un bouchon de piston pour un injecteur de médicament, dans lequel un corps de bouchon (1) est conçu avec une section d'engrènement (26,27) pour un coulisseau et au moins un élément d'étanchéité (2, 7, 20) est positionné pour le moins partiellement sur la périphérie du corps de bouchon (1), dans lequel la section d'engrènement consiste en un matériau plus mou que le corps de bouchon, **caractérisé en ce que** la section d'engrènement (26,27) est refermée par un matériau avec le corps de bouchon (1).

6. Procédé selon la revendication 5, dans lequel le corps de bouchon (1) consiste en un thermoplastique non élastomère relativement dur, en particulier du polypropylène, et la section d'engrènement (26,27) en un thermoplastique élastomère relativement mou, en particulier du santoprène.

7. Procédé selon la revendication 5 ou la revendication 6, dans lequel la section d'engrènement (26,27) est collée ou solidarisée intégralement par formage sur le corps de bouchon (1) par sa surface de contact.

8. Procédé selon la revendication 7, dans lequel la section d'engrènement (26,27) est fusionnée par sa surface de contact avec le corps de bouchon (1) ou moulée par injection en deux composants par sa surface de contact avec le corps de bouchon (1).

9. Procédé selon l'une des revendications 5-8, dans lequel le corps de bouchon (1) est d'abord moulé par injection dans sa forme finale et fixé et ensuite la section d'engrènement (26,27) est appliquée par injection, tandis que par le biais d'un réchauffement de la surface de contact il se produit un fusionnement permettant une fermeture par un matériau.

10. Procédé selon la revendication 9, dans lequel les points de fusion du thermoplastique du corps de bouchon (1) et du thermoplastique de la section d'engrènement (26,27) ne doivent pas diverger entre eux de plus de 35°C, en particulier pas plus de 20°C jusqu'à 25°C.
